# EUROPEAN PATENT APPLICATION

(11) **EP 4 730 348 A1**
(43) Date of publication of application: **22.04.2026**
(21) Application number: 25209231.7
(22) Date of filing: 16.10.2025
(51) Int. Cl.: G16H 15/00, G16H 30/00, G06Q 50/00, G06V 20/40, G16H 10/60, G16H 20/40, G16H 40/20, G16H 40/63, G06N 20/00

(54) **SYSTEM AND METHOD OF GENERATING HOSPITAL REPORTS**

(30) Priority: 18.10.2024 US 202418920489
(71) Applicant: Karl Storz SE & Co. KG, 78532 Tuttlingen (DE)
(72) Inventor: Wenzler, Sebastian, 78532 Tuttlingen (DE); Ilg, Jasmin, 78532 Tuttlingen (DE); Haag, Simon, 78532 Tuttlingen (DE); Khazatskiy, Igor, 78532 Tuttlingen (DE)

(57) **Abstract**

A system and method for generating a report of events includes receiving procedure data captured during a procedure by at least one of a first sensor and a second sensor, each of the first sensor and the second sensor communicatively coupled to data processing hardware. The system and method also include detecting a first plurality of events in the procedure data, detecting a second plurality of events in the procedure data, and for each event of the first plurality of events and the second plurality of events, associating the event with a respective timestamp of when the event occurred during the procedure. The system and method further include merging the events by synchronizing the first plurality of events and the second plurality of events based on the respective timestamps, and generating a machine readable report including the merged events.

## Description

### TECHNICAL FIELD

This disclosure relates to a system and method of generating hospital reports.

### BACKGROUND

Generally, whenever a medical procedure is performed, a comprehensive surgical report must be manually created to document the procedure after its conclusion. For example, the surgical report is a historical record of the medical procedure that may include information from the patient records, events that occurred during the procedure, the persons involved, and any possible surgical complications or incidents that occurred during the procedure. As should be appreciated, consolidating all of this data may be a tedious and time consuming task. In particular, surgeons and/or medical staff must either recall from memory the events of the procedure or review recorded video from the procedure to identify deterministic events as images that are edited together for documentation. Additionally, surgical notes, equipment documentation, and patient data must be incorporated with an image findings. In addition to being a time consuming process, this manual process is highly variable depending on the particular author of the surgical report. Moreover, due to human error, it is possible for any one of the multiple data streams to be partially incomplete or incorrectly saved. Finally, current surgical reports are created in human-readable formats, which, unlike machine-readable formats, cannot be easily analyzed.

### SUMMARY

One aspect of the disclosure provides a system for generating a report of events including a first sensor configured to capture hospital room data, a second sensor configured to capture patient data, data processing hardware communicatively coupled to the first sensor and the second sensor, and memory hardware in communication with the data processing hardware. The memory hardware stores instructions that when executed on the data processing hardware causes the data processing hardware to perform operations that include receiving procedure data captured during a procedure by at least one of the first sensor and the second sensor, detecting a first plurality of events in the procedure data captured by the first sensor, and detecting a second plurality of events in the procedure data captured by the second sensor. For each event of the first plurality of events and the second plurality of events, the operations also include associating the event with a respective timestamp of when the event occurred during the procedure. The operations also include merging the first plurality of events and the second plurality of events by synchronizing the first plurality of events and the second plurality of events based on the respective timestamps of the events, and generating a machine readable report including the merged first plurality of events and the second plurality of events.

Implementations of the disclosure may include one or more of the following optional features. In some implementations, the first sensor includes one or more of a camera or a microphone. In these implementations, detecting the first plurality of events in the procedure data captured by the first sensor may include detecting a first event in image data captured by the camera, and confirming detection of the first event by processing one of either (1) an image data captured by the second sensor and (2) audio data captured by the microphone that is synchronized with the first event. Additionally or alternatively, the first plurality of events in the procedure data captured by the first sensor may include one or more of a number of people, a classification of the people, anomalies in a sterile field, a hot surgical instrument, patient burns, a position of the people with respect to a patient, a position and number of surgical instruments, or patient position.

In some examples, the second sensor includes one or more of a camera, a microphone, or a surgical instrument. In these examples, the second plurality of events in the procedure data captured by the second sensor may include one or more of settings of the surgical instrument, a surgical instrument location, foreign material, smoke, bleeds, a type of anomaly, a location of the anomaly, vital sign changes, ventilation, or collisions. Additionally or alternatively, the surgical instrument is configured to relay instrument data indicating one or more settings of the surgical instrument to the data processing hardware.

In some implementations, merging the first plurality of events and the second plurality of events by aligning the first plurality of events and the second plurality of events based on the respective timestamps of the events includes identifying a first event in the first plurality of events, the first event captured by a camera and including image data, identifying a second event in the second plurality of events, the second event captured by a microphone, including audio data, and having a respective timestamp that is synchronized with a respective timestamp of the first event and determining that the first event and the second event conflict. In these implementations, generating the machine readable report includes omitting the second event from the machine readable report. In some examples, the operations further include generating, using a large language model configured to receive the machine readable report as input, a human readable report.

In some implementations, the operations further include receiving patient data and generating the machine readable report based on the received patient data. In some examples, the operations further include a hospital room model configured to receive the hospital room data captured by the first sensor and generate, as output, events detected in the hospital room data. In some implementations, a is patient model configured to receive the patient data captured by the second sensor and generate, as output, events detected in the patient data.

Another aspect of the disclosure provides a computer-implemented method for generating a report of events that when executed by data processing hardware causes the data processing hardware to perform operations that include receiving procedure data captured during a procedure by at least one of a first sensor and a second sensor, the first sensor configured to capture hospital room data, and the second sensor configured to capture patient data, each of the first sensor and the second sensor communicatively coupled to the data processing hardware. The operations also include detecting a first plurality of events in the procedure data captured by the first sensor, and detecting a second plurality of events in the procedure data captured by the second sensor. For each event of the first plurality of events and the second plurality of events, the operations also include associating the event with a respective timestamp of when the event occurred during the procedure. The operations also include merging the first plurality of events and the second plurality of events by synchronizing the first plurality of events and the second plurality of events based on the respective timestamps of the events, and generating a machine readable report including the merged first plurality of events and the second plurality of events.

This aspect may include one or more of the following optional features. In some implementations, the first sensor includes one or more of a camera or a microphone. In these implementations, detecting the first plurality of events in the procedure data captured by the first sensor may include detecting a first event in image data captured by the camera, and confirming detection of the first event by processing one of either (1) an image data captured by the second sensor and (2) audio data captured by the microphone that is synchronized with the first event. Additionally or alternatively, the first plurality of events in the procedure data captured by the first sensor may include one or more of a number of people, a classification of the people, anomalies in a sterile field, a hot surgical instrument, patient burns, a position of the people with respect to a patient, a position and number of surgical instruments, or patient position.

In some examples, the second sensor includes one or more of a camera, a microphone, or a surgical instrument. In these examples, the second plurality of events in the procedure data captured by the second sensor may include one or more of settings of the surgical instrument, a surgical instrument location, foreign material, smoke, fogging, a type of anomaly, a location of the anomaly, bleeds, vital sign changes, ventilation, or collisions. Additionally or alternatively, the surgical instrument is configured to relay instrument data indicating one or more settings of the surgical instrument to the data processing hardware.

In some implementations, merging the first plurality of events and the second plurality of events by aligning the first plurality of events and the second plurality of events based on the respective timestamps of the events includes identifying a first event in the first plurality of events, the first event captured by a camera and including image data, identifying a second event in the second plurality of events, the second event captured by a microphone, including audio data, and having a respective timestamp that is synchronized with a respective timestamp of the first event and determining that the first event and the second event conflict. In these implementations, generating the machine readable report includes omitting the second event from the machine readable report. In some examples, the operations further include generating, using a large language model configured to receive the machine readable report as input, a human readable report.

In some implementations, the operations further include receiving patient data and generating the machine readable report based on the received patient data. In some examples, the operations further include a hospital room model configured to receive the hospital room data captured by the first sensor and generate, as output, events detected in the hospital room data. In some implementations, a patient model configured to receive the patient data captured by the second sensor and generate, as output, events detected in the patient data.

While the foregoing implementations may illustrate optional implementations of the summarized aspects, we contemplate that these aspects may be combined individually or collectively, and that any such combination will remain fully within the scope of this disclosure. The details of one or more implementations of the disclosure are set forth in the accompanying drawings and the description below. Other aspects, features, and advantages will be apparent from the description and drawings, and from the claims.

### DESCRIPTION OF DRAWINGS

FIG. 1 is a schematic view of an example hospital report system for generating a report of events.
FIG. 2 is a schematic view of example components of the hospital report system of FIG. 1.
FIGS. 3-5 are example images including events detected by the hospital report system of FIG. 1.
FIG. 6 is a flowchart of an example arrangement of operations for a method of generating a report of events.
FIG. 7 is a schematic view of an example computing device that may be used to implement the systems and methods described herein.

Like reference symbols in the various drawings indicate like elements.

### DETAILED DESCRIPTION

Implementations herein are directed to automatically generating a machine-readable report capturing all of the events of a procedure in a highly accurate and highly repeatable manner. Moreover, the machine-readable report may incorporate audio data captured during the procedure to corroborate or corroborate and/or augment images detected during the procedure. Notably, by automatically generating the report in a machine-readable format, the report may be easily analyzed by downstream applications such as a large language model (LLM) trained to convert the machine-readable report into a human-readable report for release to the patient records.

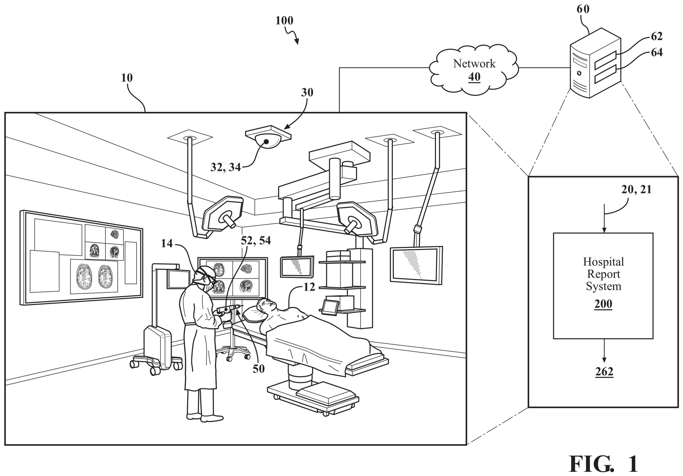

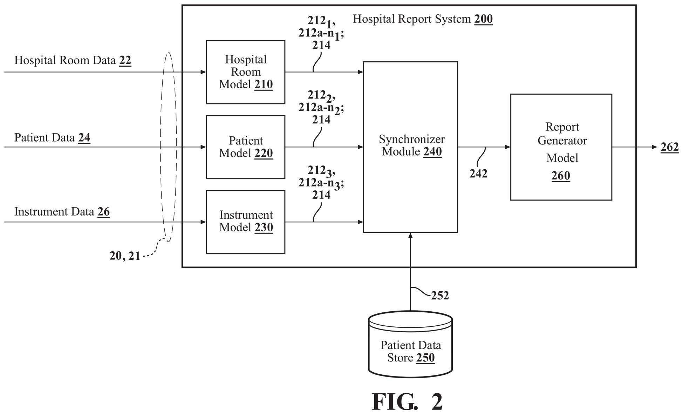

FIG. 1 illustrates an example system 100 is implemented in a procedure room 10 wherein including a patient 12 and at least one surgeon 14 performing a procedure on the patient 12. It should be appreciated that there may be other personnel to assist with the procedure, such as a nurse, an anesthesiologist, and the like. The system 100 includes a first sensor 30 and a second sensor 50 which are shown disposed within the procedure room 10, each of the first sensor 30 and the second sensor 50 are configured to capture procedure data 20. Each of the first sensor 30 and the second sensor 50 are communicatively coupled to a remote system 60 via a network 40, where the sensors 30, 50 and/or the remote system 60 execute a hospital report system 200 (FIG. 2). With reference to FIGS. 1-5, for illustrative purposes, the hospital report system 200 is provided within the context of a cholecystectomy procedure. However, it should be appreciated that the hospital report system 200 may be utilized in other surgical or procedure applications, such as, without limitation, appendectomy, colorectal surgery, hernia surgery, thyroid surgery, laryngology, esophagostomy, anesthesia, neurosurgery procedures and the like.

Briefly, and as described in further detail below, during a procedure in the procedure room 10, the hospital report system 200 is configured to receive the procedure data 20 captured in the procedure room 10, detect a first plurality of events 212₁, 212a₁-n₁ and a second plurality of events 212₂, 212a₂-n₂ in the procedure data 20, and merge the first plurality of events 212₁, 212a₁-n₁ and the second plurality of events 212₂, 212a₂-n₂ to generate a machine readable report 242 of the merged first plurality of events 212₁, 212a₁-n₁ and the second plurality of events 212₂, 212a₂-n₂. Advantageously, the machine readable report 242 is automatically generated based on the received procedure data 20, and, as such, saves significant personnel costs over existing surgical reports that are manually created. Moreover, the machine readable format of the machine readable report 242 facilitates simple analysis of the contents (e.g., events 212) captured in the machine readable report 242, as well as further processing such as, for example, conversion of the machine readable report 242 into a human readable format.

The first sensor 30 includes data processing hardware 32 and memory hardware 34 storing instructions that when executed on the data processing hardware 32 of the first sensor 30 cause the data processing hardware 32 of the first sensor 30 to perform operations. Additionally, the second sensor 50 includes data processing hardware 52 and memory hardware 54 storing instructions that when executed on the data processing hardware 52 of the second sensor 50 cause the data processing hardware 52 of the first sensor 50 to perform operations. As shown, the procedure room 10 is in communication (i.e., via the network 40) with the remote system 60 (e.g., server, cloud computing environment) that also includes data processing hardware 62 and memory hardware 64 storing instructions that when executed on the data processing hardware 62 cause the data processing hardware 62 to perform operations. For instance, the first sensor 30 and the second sensor 50 are communicatively coupled with the data processing hardware 62 of the remote system 60 such that procedure data 20 captured by the first sensor 30 and the second sensor 50 is communicated to the data processing hardware 62 executing the hospital report system 200. Advantageously, the hospital report system 200 automatically saves the procedure data 20 to minimize gaps in the procedure data 20. In some implementations, execution of the hospital report system 200 is shared across the first sensor 30, the second sensor 50, and the remote system 60.

The procedure data 20 may include corresponding temporal data 21 indicating a time that the procedure data 20 was captured. Notably, because the procedure data may come from any of the first sensor 30 and the second sensor 50, the first sensor 30 and the second sensor 50 are aligned in time such that the temporal data 21 from each of the first sensor 30 and the second sensor 50 may be aligned with respect to one another so as to be synchronized. The procedure data 20 may further include one or more of hospital room data 22, patient data 24, and instrument data 26. For instance, the first sensor 30 is configured to capture the hospital room data 22 and communicate the hospital room data 22 as procedure data 20 including corresponding temporal data 21 to the hospital report system 200. Here, the first sensor 30 may include one or more of a camera configured to capture image data (e.g., image frames) of the procedure room 10 and a microphone configured to capture audio data (e.g., acoustic frames) in the procedure room 10. As shown in FIG. 1, the first sensor 30 includes a camera system mounted in the ceiling of the procedure room 10, however the first sensor 30 may be located (i.e., mounted or freestanding) in any area of the procedure room 10 that allows the first sensor 30 to accurately capture the hospital room data 22.

Similarly, the second sensor 50 is configured to capture the patient data 24 and communicate the patient data 24 as procedure data 20 including corresponding temporal data 21 to the hospital report system 200. In particular, the second sensor 50 may include one or more of a camera configured to capture image data (e.g., image frames) of the procedure room 10, a microphone configured to capture audio data (e.g., acoustic frames) in the procedure room 10, and a surgical instrument being used in the procedure. As shown in FIG. 1, the second sensor 50 may correspond to a surgical instrument held by the surgeon 14 and used in the procedure on the patient 12. In some implementations, the surgical instrument includes an integrated camera and/or microphone to capture image data and/or acoustic data within a body cavity of the patient 12 during the procedure. Here, the integrated camera and/or microphone may capture the image data and/or audio data as patient data 24, while the surgical instrument measures one or more settings of the surgical instrument as instrument data 26, and transmits the patient data 24 together with the instrument data 26 along with the corresponding temporal data 21 for the patient data 24 and the instrument data 26. In some implementations, the instrument data 26 includes device data (also referred to as instrument data) that indicates one or more settings of a surgical instrument in the procedure. The one or more of settings of a surgical instrument may include the device data (e.g., radiofrequency (RF) generator, pump, light, etc.), light intensity, insufflation pressure and flow, and/or high frequency settings.

Referring again to FIGS. 1 and 2, the hospital report system 200 includes one or more models 210, 220, 230, a synchronizer module 240, and a report generator model 260. Additionally the hospital report system 200 has access to a patient data store 250 stored on the memory hardware 64 of the remote system 60. The one or more models 210, 220, 230 are each configured to detect respective events 212 corresponding to a respective stream of procedure data 20 captured by the at least one of the first sensor 30 and the second sensor 50 and, for each detected event 212, associate the event 212 with a respective timestamp 214 derived from the corresponding temporal data 21. For example, as shown the hospital report system includes a hospital room model 210 configured to receive the hospital room data 22 recorded by the first sensor 30, a patient model 220 configured to receive the patient data 24 recorded by the first sensor 50, and an instrument model 230 configured to receive the instrument data 26. While FIG. 2 includes three (3) models 210, 220, 230, the hospital report system 200 may include any number of additional models corresponding to additional data streams of the procedure data 20. Optionally, in instances where the second sensor 50 includes the surgical instrument, the hospital report system 200 may include only two (2) models 210, 220. In addition to detecting the respective events 212, each of the models 210, 220, 230 may be configured to detect the type of procedure being performed in the procedure room 10.

As used herein, events 212 may generally refer to changes in the procedure room 10 and/or the body cavity of the patient 12 indicating that a particular task has occurred, that a state of the procedure room 10 and/or the body cavity of the patient 12 has changed, and/or status updates from a surgical instrument (e.g., the second sensor 50). For instance, the hospital room model 210 may be configured to detect, in the received procedure data 20 (i.e., the hospital room data 22), a first plurality of events 212₁, 212a₁-n₁. Put another way, the hospital room model 210 is configured to receive, as input, the procedure data 20 and the temporal data 21 captured by the first sensor 30 and detect the first plurality of events 212₁ based on the procedure data 20. Additionally, the hospital room model 210 associates a timestamp 214 with each event 212₁ based on the corresponding temporal data 21. The first plurality of events 212₁ may include one or more of a number of people (e.g., medical staff) in the procedure room 10, classifications (e.g., surgeon, anesthesiologist, medical assistant, etc.) of the people in the procedure room 10, any anomalies (e.g. surgical instrument dropped and still used) in a surgical field of the procedure room 10, a position (e.g., prone, supine, etc.) of the patient 12, a position of the team (e.g., the medical staff) with respect to the patient 12, a hot surgical instrument, the type of procedure (e.g., appendectomy, colorectal surgery, hernia surgery, etc.), a position and number of surgical instruments, any external procedures such as draping, patient preparation, administration of anesthesia, and the like, and/or burns on the patient 12.

In implementations where the first sensor 30 includes a camera, the hospital room model 210 may include a corresponding image subsystem. Here, the hospital room model 210 receives, as input, the image data (i.e., hospital room data 22) captured by the first sensor 30, processes the image data into image frames, and performs one or more object recognition techniques (e.g., object classification, object recognition, facial recognition, etc.) on each of the image frames to detect the first plurality of events 212₁. Additionally or alternatively, when the first sensor includes a microphone, the hospital room model 210 may further include a corresponding audio subsystem. Here, the hospital room model 210 receives, as input, streaming audio data (i.e., hospital room data 22) captured by the first sensor 30 and extracts the audio data (e.g., acoustic frames) and performs acoustic detection on the acoustic frames to detect the first plurality of events 212₁. In implementations where the hospital room model 210 includes both the image subsystem and the audio subsystem (i.e., both a camera and a microphone), the image subsystem may be augmented by the audio subsystem. In other words, in instances where the image subsystem of the hospital room model 210 detects (i.e., in image frames) an event 212₁ of the first plurality of events 212₁, the audio subsystem may confirm detection of the event 212₁ by also detecting (i.e., in the acoustic frames) the event 212₁ of the first plurality of events 212₁. Here, the event 212₁ detected by the image subsystem and the event 212₁ detected by the audio subsystem of the hospital room model 210 may have respective timestamps 214 that are synchronized.

The patient model 220 may be configured to detect, in the received procedure data 20 (i.e., the patient data 24), a second plurality of events 212₂, 212a₂-n₂. Put another way, the patient model 220 is configured to receive, as input, the procedure data 20 and the temporal data 21 captured by the second sensor 50 and detect the second plurality of events 212₂ based on the procedure data 20. Additionally, the hospital room model 210 associates a timestamp 214 with each event 212₂ based on the corresponding temporal data 21. The second plurality of events 212₂ may include one or more of settings of a surgical instrument (e.g., the second sensor 50) such as device data (e.g., radio frequency (RF) generator, pump, light, etc.), light intensity, insufflation pressure and flow, and/or high frequency settings, a location of the surgical instrument (i.e., detected by a global positioning (GPS) module and/or gyroscope of the second sensor 50), a type of the surgical instrument used, and any events occurring within the body of the patient 12, such as any foreign material (e.g., consumables such as suture materials, staples, etc.) present inside or proximate to a body cavity of the patient 12, tissue detection, tumor detection, smoke levels in the body cavity of the patient 12, fogging (e.g., lens fogging), bleeds in the patient 12, a type (e.g., a tumor class) of anomaly detected in the patient 12, a location (e.g., left wall, T1) of the anomaly in the patient 12, vital sign changes of the patient 12, ventilation levels near the second sensor 50, any collisions between the second sensor 50 and the patient 12 and/or personnel (e.g., the surgeon 14), and any complications of the procedure.

In implementations where the second sensor 50 includes a camera, the patient model 220 may include a corresponding image subsystem. Here, the patient model 220 receives, as input, the image data (i.e., patient data 24) captured by the second sensor 50, processes the image data into image frames, and performs one or more object recognition techniques (e.g., object classification, object recognition, etc.) on each of the image frames to detect the second plurality of events 212₂. Additionally or alternatively, when the first sensor includes a microphone, the patient model 220 may further include a corresponding audio subsystem. Here, the patient model 220 receives, as input, streaming audio data (i.e., patient data 24) captured by the second sensor 50 and extracts the audio data (e.g., acoustic frames) and performs acoustic detection on the acoustic frames to detect the first plurality of events 212₂. In implementations where the patient model 220 includes both the image subsystem and the audio subsystem (i.e., both a camera and a microphone), the image subsystem may be augmented by the audio subsystem. In other words, in instances where the image subsystem of the patient model 220 detects (i.e., in image frames) an event 212₂ of the second plurality of events 212₂, the audio subsystem may confirm detection of the event 212₂ by also detecting (i.e., in the acoustic frames) the event 212₂ of the second plurality of events 212₂. Here, the event 212₂ detected by the image subsystem and the event 212₂ detected by the audio subsystem of the patient model 220 may have respective timestamps 214 that are synchronized.

In implementations, where the second sensor 50 includes the surgical instrument used by the surgeon 14, the instrument model 230 may be configured to detect, in the received procedure data 20 (i.e., the instrument data 26), a third plurality of events 212₃, 212a₃-n₃. Put another way, the instrument model 230 is configured to receive, as input, the procedure data 20 and the temporal data 21 captured by the second sensor 50 including the surgical instrument, and detect the third plurality of events 212₃ based on the procedure data 20. Additionally, the instrument model 230 associates a timestamp 214 with each event 212₃ based on the corresponding temporal data 21. The third plurality of events 212₃ may include one or more of settings of a surgical device, such as insufflator settings (heated/humidified gas), hose settings, etc., mode and activation duration settings such as pump, medication administration, etc., a classification of the surgical instrument, motor data of the surgical instrument, and smoke extraction levels by the surgical device.

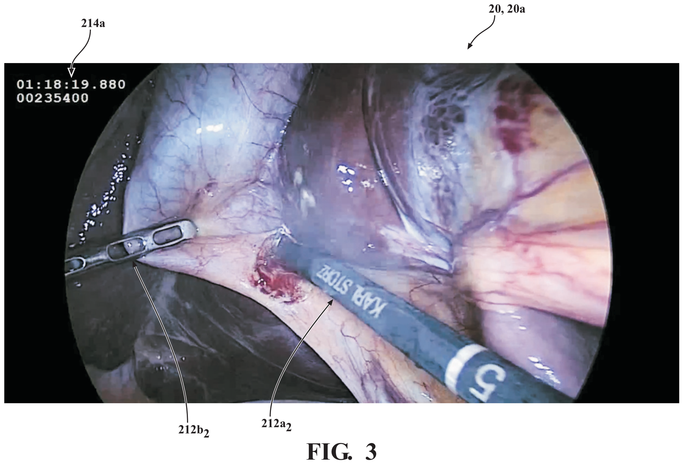

Referring briefly to FIGS. 3-5, example images including events 212 detected by the hospital report system 200 are shown. Here, and as noted above, the hospital report system 200 may automatically generate a machine readable report 242 based on the events 212 detected in procedure data 20 from a cholecystectomy procedure. As shown, the procedure data 20a-20c (i.e., frames from image data) may be detected by a camera in the second sensor 50. Here, the camera (i.e., the second sensor 50) may be integrated with an endoscope used to record the cholecystectomy procedure, or be a separate system from the endoscope.

With particular reference to FIG. 3, the procedure data 20a includes a frame of the interior body cavity of the patient 12 during the cholecystectomy procedure. Here, the patient model 220 may receive the procedure data 20a (i.e., patient data 24) as input, and, using an image subsystem of the patient model 220, detect that the procedure data 20a includes one or more events 212. For example, as shown, the patient model 220 detects that the procedure data 20a may include the event 212a₂ of a coagulation instrument present and the event 212b₂ of a grasper present. The patient model 220 may further associate the timestamp 214a with each of the detected events 212a₂ and 212b₂.

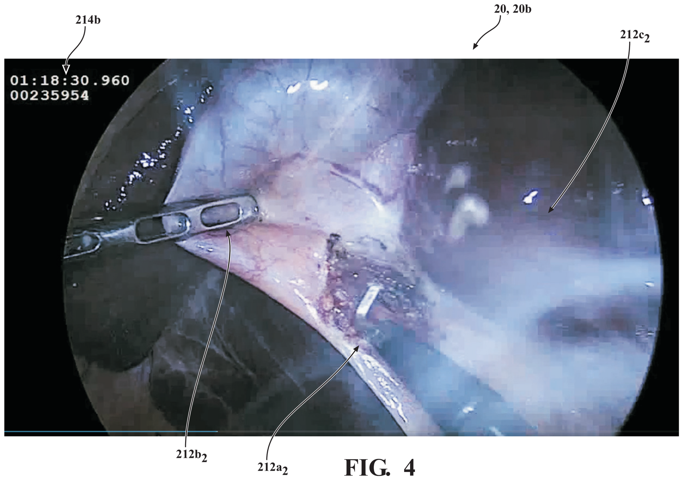

Referring to FIG. 4, as the cholecystectomy procedure continues, the procedure data 20b captured by the second sensor 50 may include an additional detected event 212. Here, the patient model 220 receives the procedure data 20b (i.e., the patient data 24) as input, and, using the image subsystem of the patient model 220, detects events 212 in the procedure data 20b. For example, as shown, the patient model 220 detects that the procedure data 20b includes the event 212a₂ of the coagulation instrument present and the event 212b₂ of the grasper present. Additionally, the patient model 220 detects the event 212c₂ of increased smoke present. For example, the patient model 220 may compare the smoke level in the procedure data 20b with previous image frames of procedure data (e.g., procedure data 20a) including a smoke level, and detect the event 212c₂ of increased smoke present when the change in smoke levels between the procedure data 20b and previous procedure data exceeds a threshold change level. The patient model 220 may further associate the timestamp 214b with each of the detected events 212a₂, 212b₂, 212c₂.

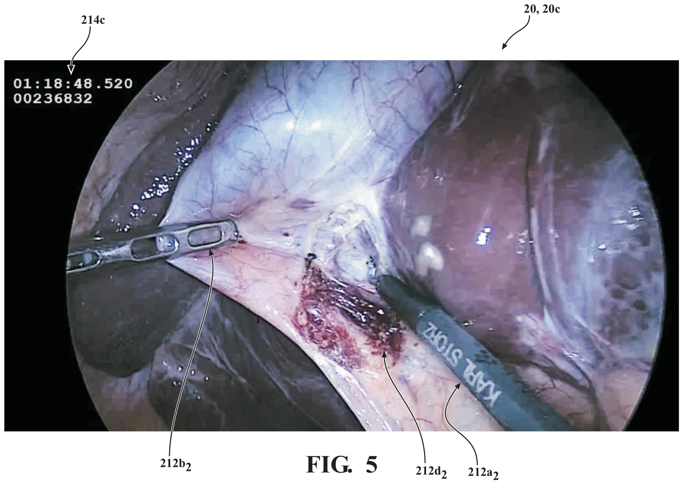

Referring to FIG. 5, as the cholecystectomy procedure continues, the procedure data 20c captured by the second sensor 50 may include an additional detected event 212. Here, the patient model 220 receives the procedure data 20c (i.e., the patient data 24) as input, and, using the image subsystem of the patient model 220, detects events 212 in the procedure data 20c. For example, as shown, the patient model 220 detects that the procedure data 20c includes the event 212a₂ of the coagulation instrument present and the event 212b₂ of the grasper present. Additionally, the patient model 220 detects the event 212d₂ of increased bleeding in the patient 12. For example, the patient model 220 may compare the bleed level in the procedure data 20c with previous image frames of procedure data (e.g., procedure data 20a, 20b) including a bleed level, and detect the event 212d₂ of increased bleeding present when the change in bleed levels between the procedure data 20c and previous procedure data exceeds a threshold change level. Notably, as the smoke level has decreased, the patient model 220 no longer detects the event 212 of increased smoke present. The patient model 220 may further associate the timestamp 214c with each of the detected events 212a₂, 212b₂, 212d₂.

Referring again to FIG. 2, the synchronizer module 240 is configured to receive the plurality of events 212 (e.g., the first plurality of events 212₁, the second plurality of events 212₂, and/or the third plurality of events 212₃) and the respective timestamps 214 of each event 212 as input, and generate, as output, a machine readable report 242 of plurality of events 212. For instance, the synchronizer module 240 may merge the first plurality of events 212₁ and the second plurality of events 212₂ by synchronizing the first plurality of events 212₁ and the second plurality of events 212₂ by aligning the first plurality of events 212₁ and the second plurality of events 212₂ based on the respective timestamps 214. Here, merging may refer to the synchronizer module 240 generating an ordered list based on the respective timestamps 214 in chronological order. Where, when more than one event 212 has a same respective timestamp 214, the synchronizer module 240 may reconcile the more than one event 212 to either confirm or omit one or more of the more than one events 212 from the machine readable report 232. It should be appreciated that, by the synchronizer module 240 automatically merging the first plurality of events 212₁ and the second plurality of events 212₂ based upon a known medical procedure, processing time is greatly reduced for confirming the events 212. Notably, the synchronizer module 240 may merge any number of plurality of events 212 based on the number of input data streams that form the procedure data 20. The machine readable report 232 generated by the synchronizer module 240 may include an xml format, a txt format, or any other machine readable format.

In some implementations, the synchronizer module 240 may generate the machine readable report 242 as a merged list of all of the detected events 212 in order of the respective timestamps 214 of the detected events 212. When merging the first plurality of events 212₁ and the second plurality of events 212₂, the synchronizer module 240 may identify two or more detected events 212 in the first plurality of events 212₁ and the second plurality of events 212₂ that are synchronized (i.e., have a same timestamp 214). For example, the synchronizer module 240 may determine that the two more detected events 212 correlate, or determine that the two or more detected events 212 conflict. In instances where the synchronizer module 240 receives a first detected event 212 in image data captured by a camera (e.g., the first sensor 30) and a detected event 212 in audio data captured by a microphone (e.g., the first sensor 30), and determines, based on the respective timestamps 214, that the detected event 212 in audio data is synchronized with the detected first event 212, the synchronizer module 240 may confirm the detection of the event 212. Similarly, the synchronizer module 240 may confirm the detection of the event 212 based on detecting an event 212 in image data captured by a camera of the second sensor 50.

Continuing with the example, the synchronizer module 240 may receive a first event 212 from the hospital room model 210 detected in image data captured by the first sensor 30, the first event 212 including smoke. Here, if the synchronizer module 240 identifies that the hospital room model 210 further detects a second event 212 in audio data, the second event 212 including acoustic data corresponding to the actuation of an instrument and having a timestamp 214 corresponding to the timestamp 214 of the first event 212, the synchronizer module 240 may confirm detection of the event 212 as the actuation of a surgical instrument to cauterize a portion of the body cavity of the patient 12. Likewise, if the synchronizer module 240 identifies that the patient model 220 further detects a third event 212 in image data, the third event 212 including image data captured by the second sensor 50, including smoke, and having a timestamp 214 corresponding to the timestamp 214 of the first event 212, the synchronizer module 240 may confirm detection of the event 212 as the actuation of a surgical instrument to cauterize a portion of the body cavity of the patient 12.

Conversely, the synchronizer module 240 may compare conflicting events 212 detected by the models 210, 220, 230 that occur at the same time (i.e., based on the respective timestamps 214) and omit one or more of the conflicting events 212 from the machine readable report 242. For example, the synchronizer module 240 may identify a first event 212₁ of the first plurality of events 212₁ detected by the hospital room model 210 and a second event 212₂ of the second plurality of events 212₂. Here, the first event may include image data of the procedure room 10 captured by the first sensor 30, the image data including an event that the surgeon 14 is present and holding the surgical instrument away from the patient 12. The second event 212₂ may include audio data captured by a microphone of the second sensor 50, and have a respective timestamp 214 that is synchronized with the first event 212₁. If the second event 212₂ conflicts with the first event 212₁, such as including audio data including a burning sound when the first event 212₁ clearly shows the surgical instrument is not activated, the synchronizer module 240 may disregard the second event 212₂ when merging the first plurality of events 212₁ and the second plurality of events 212₂ by omitting the second event 212₂ from the machine readable report 242. Similarly, the synchronizer module 240 may omit and/or disregard detected events 212 when the detected events 212 conflict with the particular procedure (e.g., the cholecystectomy). For example, if the synchronizer module 240 receives a detected event 212 of dilator during a cholecystectomy procedure, where a dilator would not be used, the synchronizer module 240 may disregard the detected event 212 of the dilator when generating the machine readable report 242. In this example, patient data 252 of the patient 12 may include a record indicating that the patient 12 is undergoing the cholecystectomy procedure.

In some implementations, the machine readable report 242 is based on patient data 252. In particular, the patient data store 250 may store patient data 252 (i.e., medical records) for the patient 12. The patient data 252 may include, without limitation, personally identifiable information of the patient 12, a diagnosis of the patient 12, previous medical history of the patient 12 such as previous illnesses or procedures, the body mass index (BMI) of the patient 12, age of the patient 12, the sex of the patient 12, preliminary examinations, or administrations of particular medications (e.g., tumor markers such as indocyanine green (ICG), etc.) before the procedure. As shown in FIG. 2, the synchronizer module 240 receives the patient data 252 from the patient data store, the patient data 252 associated with the particular patient 12 undergoing the cholecystectomy procedure. Thereafter, the synchronizer module 240 appends the patient data 252 to the merged list of events 212 to generate the machine readable report 242.

The report generator model 260 may receive the machine readable report 242 from the synchronizer module 240 as input, and generate, as output, a human readable report 262 including the merged list of the plurality of events 212 and the patient data 252. The human readable report 262 may generally approximate the traditional surgical reports that are manually produced after each procedure, but is generated automatically by the hospital report system 200, thereby saving time and resources while also increasing the accuracy and repeatability of the traditional surgical report. The report generator model 260 may include a large language mode (LLM) trained to generate human readable reports 262 by incorporating the machine readable report 242 into a standard surgical report template. While commercial off-the-shelf LLMs (e.g., ChatGPT, Jasper, Neuroflash, etc.) may be poorly suited for generating these specialized surgical reports that include the lengthy structured machine readable report 242 incorporating the events 212 of the procedure and the patient data 252, the report generator model 260 may be trained (e.g., via fine-tuning) to provide the personalized human readable report 262 in response to being fed a prompt (e.g., the machine readable report 242). Additionally, during inference, the report generator model 260 (i.e., the LLM) may perform reinforcement learning that further fine-tunes the LLM on what system settings lead to the best surgical outcomes. For instance, a prior human readable report 262 may have included an event 212 of a tumor and recommended borders of the tumor based on a certain position of the tumor. Here, the LLM may receive post operative data indicating recurrence of the tumor, and may fine-tune its parameters to define wider borders for the next procedure.

In some implementations, the report generator model 260 is further trained to confirm the accuracy of the merged list of events 212 generated by the synchronizer module 240. For example, the report generator model 260 may additionally receive the procedure data 20 and the detected first plurality of events 212₁ and detected second plurality of events 212₂ and verify that the events 212 included in the merged list of events 212 is accurate. In other words, the report generator model 260 may act as the final arbiter of the procedure data 20 by ensuring the synchronizer module 240 has applied the correct weights and/or deference to the events detected by the models 210, 220, 230. In some implementations, where the report generator model 260 detects an inconsistency or inaccuracy between synchronized events 212 in the procedure data 20, the report generator model 260 may update the merged list of events 212 by either adding, omitting, and/or revising events 212 in the merged list of events 212. Here, the report generator model 260 may generate the human readable report 262 based on the updated merged list of events 212.

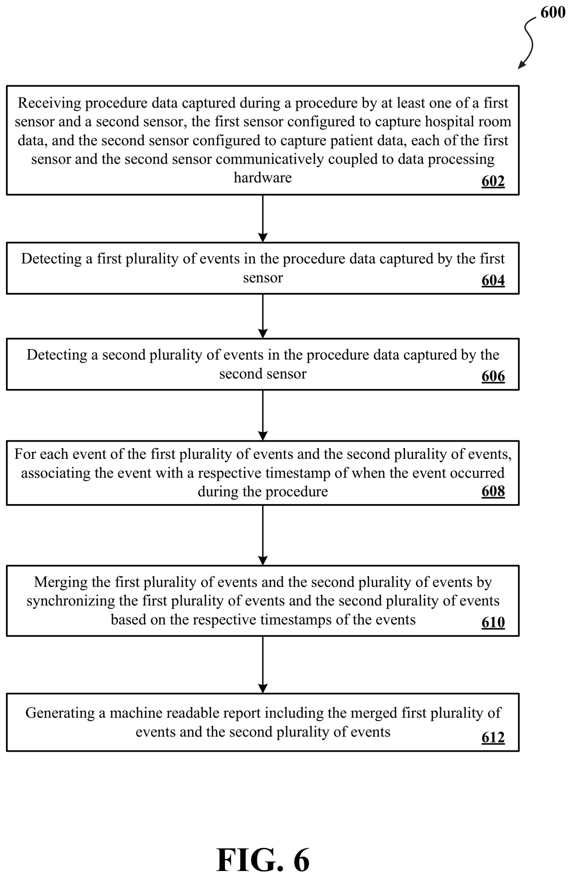

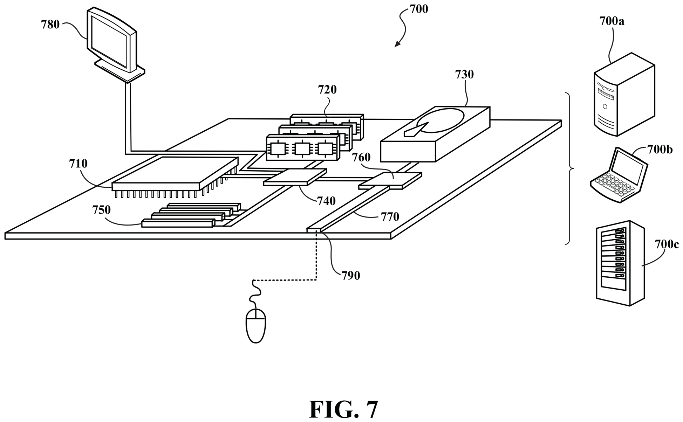

FIG. 6 shows a flowchart of an example arrangement of operations for a method 600 of generating a report of events 212. The method may be described with reference to FIGS. 1-5. Data processing hardware (e.g., data processing hardware 62 of FIG. 1; data processing hardware 910 of FIG. 7) may execute instructions stored on memory hardware (e.g., memory hardware 64 of FIG 1; memory hardware 920 of FIG. 7) to perform the example arrangement of operations for the method 600.

The method 600 includes, at operation 602, receiving procedure data 20 captured during a procedure by at least one of a first sensor 30 and a second sensor 50. Here, the first sensor 30 is configured to capture hospital room data, and the second sensor 50 is configured to capture patient data, where each of the first sensor 30 and the second sensor 50 are communicatively coupled to data processing hardware 62. The method 600 also includes, at operation 604, detecting a first plurality of events 212₁, 2121a-n₁ in the procedure data 20 captured by the first sensor 30. At operation 606, the method 600 also includes detecting a second plurality of events 212₂, 2121a-n₂ in the procedure data 20 captured by the second sensor 50.

For each event 212 of the first plurality of events 212₁ and the second plurality of events 212₂, the method 600 further includes, at operation 608, associating the event 212 with a respective timestamp 214 of when the event 212 occurred during the procedure. At operation 610, the method 600 also includes merging the first plurality of events 212₁ and the second plurality of events 212₂ by synchronizing the first plurality of events 212₁ and the second plurality of events 212₂ based on the respective timestamps 214 of the events 212. The method 600 also includes, at operation 612, generating a machine readable report 242 including the merged first plurality of events 212₁ and the second plurality of events 212₂

FIG. 7 is schematic view of an example computing device 700 that may be used to implement the systems and methods described in this document. The computing device 700 is intended to represent various forms of digital computers, such as laptops, desktops, workstations, personal digital assistants, servers, blade servers, mainframes, and other appropriate computers. The components shown here, their connections and relationships, and their functions, are meant to be exemplary only, and are not meant to limit implementations of the inventions described and/or claimed in this document.

The computing device 700 includes a processor 710, memory 720, a storage device 730, a high-speed interface/controller 740 connecting to the memory 720 and high-speed expansion ports 750, and a low speed interface/controller 760 connecting to a low speed bus 770 and a storage device 730. Each of the components 710, 720, 730, 740, 750, and 760, are interconnected using various busses, and may be mounted on a common motherboard or in other manners as appropriate. The processor 710 (e.g., the data processing hardware 62 of FIG. 1) can process instructions for execution within the computing device 700, including instructions stored in the memory 720 or on the storage device 730 to display graphical information for a graphical user interface (GUI) on an external input/output device, such as display 780 coupled to high speed interface 740. In other implementations, multiple processors and/or multiple buses may be used, as appropriate, along with multiple memories and types of memory. Also, multiple computing devices 700 may be connected, with each device providing portions of the necessary operations (e.g., as a server bank, a group of blade servers, or a multi-processor system).

The memory 720 (e.g., the memory hardware 64 of FIG. 1) stores information non-transitorily within the computing device 700. The memory 720 may be a computer-readable medium, a volatile memory unit(s), or non-volatile memory unit(s). The non-transitory memory 720 may be physical devices used to store programs (e.g., sequences of instructions) or data (e.g., program state information) on a temporary or permanent basis for use by the computing device 700. Examples of non-volatile memory include, but are not limited to, flash memory and read-only memory (ROM) / programmable read-only memory (PROM) / erasable programmable read-only memory (EPROM) / electronically erasable programmable read-only memory (EEPROM) (e.g., typically used for firmware, such as boot programs). Examples of volatile memory include, but are not limited to, random access memory (RAM), dynamic random access memory (DRAM), static random access memory (SRAM), phase change memory (PCM) as well as disks or tapes.

The storage device 730 is capable of providing mass storage for the computing device 700. In some implementations, the storage device 730 is a computer-readable medium. In various different implementations, the storage device 730 may be a floppy disk device, a hard disk device, an optical disk device, or a tape device, a flash memory or other similar solid state memory device, or an array of devices, including devices in a storage area network or other configurations. In additional implementations, a computer program product is tangibly embodied in an information carrier. The computer program product contains instructions that, when executed, perform one or more methods, such as those described above. The information carrier is a computer- or machine-readable medium, such as the memory 720, the storage device 730, or memory on processor 710.

The high speed controller 740 manages bandwidth-intensive operations for the computing device 700, while the low speed controller 760 manages lower bandwidth-intensive operations. Such allocation of duties is exemplary only. In some implementations, the high-speed controller 740 is coupled to the memory 720, the display 780 (e.g., through a graphics processor or accelerator), and to the high-speed expansion ports 750, which may accept various expansion cards (not shown). In some implementations, the low-speed controller 760 is coupled to the storage device 730 and a low-speed expansion port 790. The low-speed expansion port 790, which may include various communication ports (e.g., USB, Bluetooth, Ethernet, wireless Ethernet), may be coupled to one or more input/output devices, such as a keyboard, a pointing device, a scanner, or a networking device such as a switch or router, e.g., through a network adapter.

The computing device 700 may be implemented in a number of different forms, as shown in the figure. For example, it may be implemented as a standard server 700a or multiple times in a group of such servers 700a, as a laptop computer 700b, or as part of a rack server system 700c.

Various implementations of the systems and techniques described herein can be realized in digital electronic and/or optical circuitry, integrated circuitry, specially designed ASICs (application specific integrated circuits), computer hardware, firmware, software, and/or combinations thereof. These various implementations can include implementation in one or more computer programs that are executable and/or interpretable on a programmable system including at least one programmable processor, which may be special or general purpose, coupled to receive data and instructions from, and to transmit data and instructions to, a storage system, at least one input device, and at least one output device.

A software application (i.e., a software resource) may refer to computer software that causes a computing device to perform a task. In some examples, a software application may be referred to as an "application," an "app," or a "program." Example applications include, but are not limited to, system diagnostic applications, system management applications, system maintenance applications, word processing applications, spreadsheet applications, messaging applications, media streaming applications, social networking applications, and gaming applications.

The non-transitory memory may be physical devices used to store programs (e.g., sequences of instructions) or data (e.g., program state information) on a temporary or permanent basis for use by a computing device. The non-transitory memory may be volatile and/or non-volatile addressable semiconductor memory. Examples of non-volatile memory include, but are not limited to, flash memory and read-only memory (ROM) / programmable read-only memory (PROM) / erasable programmable read-only memory (EPROM) / electronically erasable programmable read-only memory (EEPROM) (e.g., typically used for firmware, such as boot programs). Examples of volatile memory include, but are not limited to, random access memory (RAM), dynamic random access memory (DRAM), static random access memory (SRAM), phase change memory (PCM) as well as disks or tapes.

These computer programs (also known as programs, software, software applications or code) include machine instructions for a programmable processor, and can be implemented in a high-level procedural and/or object-oriented programming language, and/or in assembly/machine language. As used herein, the terms "machine-readable medium" and "computer-readable medium" refer to any computer program product, non-transitory computer readable medium, apparatus and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The term "machine-readable signal" refers to any signal used to provide machine instructions and/or data to a programmable processor.

The processes and logic flows described in this specification can be performed by one or more programmable processors, also referred to as data processing hardware, executing one or more computer programs to perform functions by operating on input data and generating output. The processes and logic flows can also be performed by special purpose logic circuitry, e.g., an FPGA (field programmable gate array) or an ASIC (application specific integrated circuit). Processors suitable for the execution of a computer program include, by way of example, both general and special purpose microprocessors, and any one or more processors of any kind of digital computer. Generally, a processor will receive instructions and data from a read only memory or a random access memory or both. The essential elements of a computer are a processor for performing instructions and one or more memory devices for storing instructions and data. Generally, a computer will also include, or be operatively coupled to receive data from or transfer data to, or both, one or more mass storage devices for storing data, e.g., magnetic, magneto optical disks, or optical disks. However, a computer need not have such devices. Computer readable media suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto optical disks; and CD ROM and DVD-ROM disks. The processor and the memory can be supplemented by, or incorporated in, special purpose logic circuitry.

To provide for interaction with a user, one or more aspects of the disclosure can be implemented on a computer having a display device, e.g., a CRT (cathode ray tube), LCD (liquid crystal display) monitor, or touch screen for displaying information to the user and optionally a keyboard and a pointing device, e.g., a mouse or a trackball, by which the user can provide input to the computer. Other kinds of devices can be used to provide interaction with a user as well; for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's client device in response to requests received from the web browser.

A number of implementations have been described. Nevertheless, it will be understood that various modifications may be made without departing from the spirit and scope of the disclosure. Accordingly, other implementations are within the scope of the following claims.

## Claims

1. A system (200) for generating a report of events, the system comprising:
a first sensor (30) configured to capture hospital room data (22);
a second sensor (50) configured to capture patient data (24);
data processing hardware (62) communicatively coupled to the first sensor (30) and the second sensor (50); and
memory hardware (64) in communication with the data processing hardware (62) and storing instructions that when executed on the data processing hardware (62) cause the data processing hardware (62) to perform operations comprising:
receiving procedure data (20) captured during a procedure by at least one of the first sensor (30) and the second sensor (50);
detecting a first plurality of events (212₁, 212a₁-n₁ ) in the procedure data (20) captured by the first sensor (30);
detecting a second plurality of events (212₂, 212a₂-n₂) in the procedure data captured (20) by the second sensor (50);
for each event of the first plurality of events (212₁, 212a₁-n₁ ) and the second plurality of events (212₂, 212a₂-n₂), associating the event with a respective timestamp (214) of when the event occurred during the procedure;
merging the first plurality of events (212₁, 212a₁-n₁ ) and the second plurality of events (212₂, 212a₂-n₂) by synchronizing the first plurality of events (212₁, 212a₁-n₁) and the second plurality of events (212₂, 212a₂-n₂ ) based on the respective timestamps (214) of the events; and
generating a machine readable report (242) including the merged first plurality of events (212₁, 212a₁-n₁) and the second plurality of events (212₂, 212a₂-n₂).

2. The system of claim 1, wherein the first sensor (30) comprises one or more of:
a camera; or
a microphone.

3. The system of claim 2, wherein detecting the first plurality of events (212₁, 212a₁-n₁ ) in the procedure data (20) captured by the first sensor (30) comprises:
detecting a first event (212₁) in image data captured by the camera; and
confirming detection of the first event (212₁) by processing one of either (1) an image data captured by the second sensor (50) and (2) audio data captured by the microphone that is synchronized with the first event (212₁).

4. The system of claim 1, wherein the second sensor (50) comprises one or more of:
a camera;
a microphone; or
a surgical instrument configured to relay instrument data indicating one or more settings of the surgical instrument to the data processing hardware (62).

5. The system of claim 1, wherein merging the first plurality (212₁, 212a₁-n₁) of events and the second plurality of events (212₂, 212a₂-n₂) by aligning the first plurality of events (212₁, 212a₁-n₁) and the second plurality of events (212₂, 212a₂-n₂) based on the respective timestamps of the events comprises:
identifying a first event (212₁) in the first plurality of events (212₁, 212a₁-n₁ ), the first event (212₁) captured by a camera and including image data;
identifying a second event (212₂ ) in the second plurality of events (212₂, 212a₂-n₂), the second event (212₂ ) captured by a microphone, including audio data, and having a respective timestamp that is synchronized with a respective timestamp of the first event (212₁ ); and
determining that the first event (212₁ ) and the second event (212₂ ) conflict.

6. The system of claim 1, wherein the operations further comprise generating, using a large language model configured to receive the machine readable report (242) as input, a human readable report.

7. The system of claim 1, wherein the operations further comprise receiving patient data (252) and generating the machine readable report (242) based on the received patient data (252).

8. The system of claim 1, further comprising a hospital room model configured to receive the hospital room data (22) captured by the first sensor (30) and generate, as output, events detected in the hospital room data (22).

9. The system of claim 1, further comprising a patient model configured to receive the patient data (252) captured by the second sensor (50) and generate, as output, events detected in the patient data (252).

10. A computer-implemented method for generating a report of events, that when executed on data processing hardware (62), causes the data processing hardware (62) to perform operations comprising:
receiving procedure data (20) captured during a procedure by at least one of a first sensor (30) and a second sensor(50), the first sensor (30) configured to capture hospital room data (22), and the second sensor (50) configured to capture patient data (252), each of the first sensor and the second sensor communicatively coupled to the data processing hardware (62);
detecting a first plurality of events (212₁, 212a₁-n₁ ) in the procedure data (20) captured by the first sensor (30);
detecting a second plurality of events (212₂, 212a₂-n₂) in the procedure data (20) captured by the second sensor (50);
for each event of the first plurality of events (212₁, 212a₁-n₁ ) and the second plurality of events (212₂, 212a₂-n₂ ), associating the event with a respective timestamp of when the event occurred during the procedure;
merging the first plurality of events (212₁, 212a₁-n₁ ) and the second plurality of events by synchronizing the first plurality of events (212₁, 212a₁-n₁ ) and the second plurality of events (212₂, 212a₂-n₂ ) based on the respective timestamps of the events; and
generating a machine readable report (242) including the merged first plurality of events (212₁, 212a₁-n₁ ) and the second plurality of events (212₂, 212a₂-n₂).

11. The method of claim 10, wherein the first sensor (30) comprises one or more of:
a camera; or
a microphone.

12. The method of claim 10, wherein detecting the first plurality of events (212₁, 212a₁-n₁ ) in the procedure data (20) captured by the first sensor (30) comprises:
detecting a first event (212₁ ) in image data captured by the camera; and
confirming detection of the first event (212₁ ) by processing one of either (1) an image data captured by the second sensor (50) and (2) audio data captured by the microphone that is synchronized with the first event (212₁ ).

13. The method of claim 11, wherein the second sensor (50) comprises one or more of:
a camera;
a microphone; or
a surgical instrument configured to relay instrument data (26) indicating one or more settings of the surgical instrument (26) to the data processing hardware (62).

14. The method of claim 10, wherein merging the first plurality of events (212₁, 212a₁-n₁ ) and the second plurality of events (212₂, 212a₂-n₂ ) by aligning the first plurality of events (212₁, 212a₁-n₁ ) and the second plurality of events (212₂, 212a₂-n₂ ) based on the respective timestamps (214) of the events comprises:
identifying a first event (212₁ ) in the first plurality of events (212₁, 212a₁-n₁ ), the first event (212₁) captured by a camera and including image data;
identifying a second event (212₂ ) in the second plurality of events (212₂, 212a₂-n₂), the second event (212₂ ) captured by a microphone, including audio data, and having a respective timestamp (214) that is synchronized with a respective timestamp of the first event (212₁ ); and
determining that the first event (212₁ ) and the second event (212₂) conflict.

15. The method of claim 10, wherein the operations further comprise generating, using a large language model configured to receive the machine readable report (242) as input, a human readable report (262).

16. The method of claim 10, wherein the operations further comprise receiving patient data (252) and generating the machine readable report (242) based on the received patient data (252).

17. The method of claim 10, further comprising a hospital room model configured to receive the hospital room data (22) captured by the first sensor (30) and generate, as output, events detected in the hospital room data (22).
